# EUROPEAN PATENT SPECIFICATION

(11) **EP 0 526 986 B1**
(45) Date of publication and mention of the grant of the patent: **06.05.1998**
(21) Application number: 92306148.5
(22) Date of filing: 03.07.1992
(51) Int. Cl.: A61M 36/00, A61M 5/42

(54) **Injector for the insertion of an object into part of the body**
Injektor zum Einführen eines Gegenstandes in einen Körperteil
Injecteur pour introduire un objet dans une partie du corps

(30) Priority: 05.07.1991 NL 9101183
(43) Date of publication of application: 10.02.1993
(73) Proprietor: TEXAS INSTRUMENTS INCORPORATED, Dallas Texas 75265 (US); TEXAS INSTRUMENTS HOLLAND B.V., 7602 EM Almelo (NL)
(72) Inventor: Vogel, Lorrie G., Dallas, Texas 75214 (US)
(74) Representative: Schwepfinger, Karl-Heinz, Dipl.-Ing.

(56) References cited:
- NL-A- 8 502 609
- NL-A- 8 901 004
- US-A- 3 400 715

## Description

The present invention relates to an injector for the insertion of an object such as a transponder into a part of the body, of the type defined in the precharacterizing part of claim 1.

Such an injector is generally known in the state of the art. A transponder is placed with an injector in the head of, for example, a pig with the aid of, for example, a stop. The injector is in this case manipulated in such a way relative to a particular part of the animal, for example the base of the ear, that correct insertion of the object such as a transponder is achieved.

In the document NL-A-8901004 an injector is disclosed which comprises a guide means the lower surface of which has a form which corresponds to the part of the body into which the object is to be inserted.

Although such an injector and the manner of injecting are adequate for the insertion of transponders in parts of the body where the depth is of minor importance, problems occur if attempts are made to inject transponders into a thin and supple part such as an ear flap. In that situation it is virtually impossible to position the injector correctly relative to said part of the body. Besides, in the case of thin parts of the body there is the problem that the position of an object has to be defined very accurately, otherwise it cannot be guaranteed sufficiently that the transponder will be inserted correctly.

The object of the invention is to avoid these disadvantages.

This object is achieved by an injector of the type described above including the features of the characterizing part of claim 1. In particular, the guide means comprises means which shape and position said supple part of the body.

The invention is based on the idea of positioning and shaping the part of the body relative to the injector, rather than positioning the injector relative to the part of the body. The supple parts of the body can comprise all supple parts known in animals, such as ear flaps, the space between the lower jaw rami, and folds in the skin. The guide means comprise a support with a surface, which support is designed to rest against the supple part of the body, while said surface is composed of two substantially parallel parts lying at a distance from each other, between which a transition part is provided, the piercing device for the supple part of the body being inserted near said transition part.

If a "kink" is also provided in said part of the body through the surface of the support, an object can be inserted in a particularly simple manner at the place of the kink, which object can subsequently be positioned in the thin, supple part of the body. In this way, easier insertion of the object is achieved, on the one hand, while it is ensured, on the other, that the transponder is situated in the correct position.

In order to improve and facilitate abutment of the supple part of the body against the surface of the support, the injector is provided with auxiliary guide means comprising an auxiliary support with an auxiliary surface of a shape corresponding to the shape of the surface of the support, and fitted in such a way on the injector that in the operating position the supple part of the body is confined between the surface of the support and the auxiliary surface of the auxiliary support. The supple part of the body is in fact wedged between the support and the auxiliary support.

Although the support and the auxiliary support are or can be connected to each other in any manner known in the art, it is preferable for them to be hinged to each other. Furthermore, it is preferable for means to be present for exerting a force in order to move the support and the auxiliary support towards each other for the purpose of confining the supple part of the body.

According to an advantageous embodiment of the invention, the object to be inserted comprises the piercing device described above. Contamination can be prevented as far as possible in this way. The injector can comprise all injectors known in the art or parts of known injectors.

As described above, the essence of the invention is that the injector is no longer positioned relative to the part of the body.

By using the present injector, the object may be fitted in the ear flap of an animal and in particular is fitted at the lowerside of the cartilage of the earflap. Placing under this cartilage-type material ensures optimum protection of the object once placed in the ear flap of the animal in question.

The invention will be explained in greater detail below with reference to examples of embodiments shown in the drawing, in which:
Fig. 1 shows in perspective a first embodiment of the invention;
Fig. 2 shows the injector according to Fig. 1 in the position at the time of and after insertion;
Fig. 3 shows the position at the time of insertion of an object into the supple part of the body;
Fig. 4 shows the position after insertion into the supple part of the body;
Fig. 5 shows a further embodiment of the injector according to the invention.

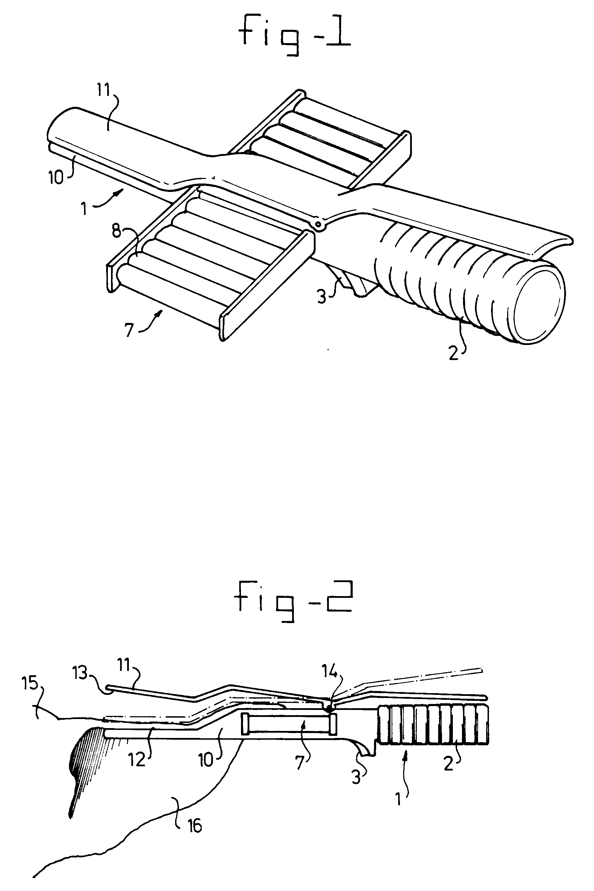

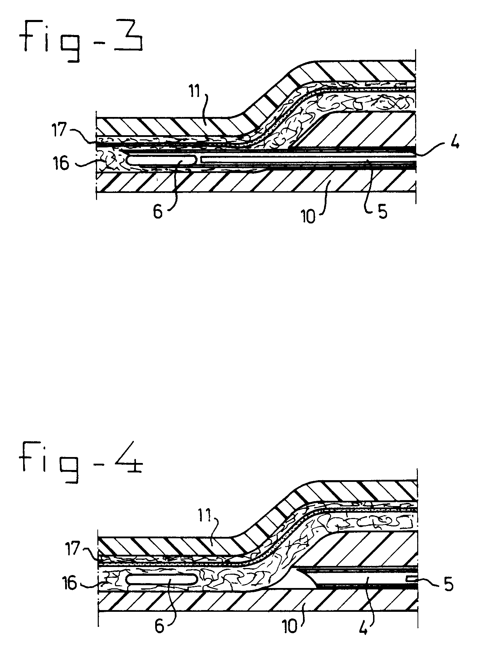

In Fig. 1 the injector according to the invention is indicated in its entirety by 1. It comprises a grip 2 with actuation means 3 for moving forward, in a manner which is known in the state of the art, a needle 4 shown in Fig. 3 and a push rod 5 for moving a transponder 6. Such a transponder is stored in a cartridge 7. The cavities 8 for transponders can in this case be sealed off on both sides and, in addition to the transponder, can be filled with a disinfectant. When the needle 4 and push rod 5 pass through the cartridge, both transponder 6 and disinfectant are moved forward.

Injector 1 also comprises a support 10 and an auxiliary support 11. It can be seen from Fig. 2 that support 10 is provided with a support surface 12, while auxiliary support 11 is provided with an auxiliary surface 13. Auxiliary support 11 is hinged at 14 to support 10. Spring devices, which are not shown, are present in order to drive auxiliary support 11 into the closed position shown in Fig. 1.

Fig. 2 shows a part 15 of the pig's head, more particularly the supple part of the body forming the ear flap 16. In Fig. 2 a solid line indicates how injector 1 is positioned relative to said ear flap 16. Then through no further operation, auxiliary support 11 is moved to support 10, as a result of which a part of ear flap 16 is confined between surface 12 of support 10 and auxiliary surface 13 of auxiliary support 11 (dashed line in Fig. 2).

This is shown in more detail in Fig. 3. It can be seen here that the ear is composed of connective tissue with a cartilage-type tissue 16 being present in the centre. It can be seen from Fig. 3 that, after the placing of the injector 1 according to Fig. 3, through operation of actuation means 3 needle 4 is moved forward and enters ear flap 16 under the cartilage; transponder 6 is then simultaneously moved forward and placed in the desired position. This is shown in Fig. 4, where the position is shown after the withdrawal of push rod 5 and needle 4. The front end of needle 4 touches the part of the body as high up as possible, in order to prevent it from slipping.

Although in this embodiment conveyance of the transponder in the supple part of the body takes place with the aid of a needle and push rod, it is also possible "to shoot" the transponder into the supple part of the body. For this purpose, the transponder is provided with piercing devices such as a pointed end. Contamination by the needle and the push rod in the tissue of the animal is thereby avoided, so that infection can be prevented.

Fitting the transponder under the cartilage 16 ensures that it is protected as well as possible. If the ear flap is relatively thin relative to the thickness of the transponder, a local thickness, which will disappear after some time, will occur. Not piercing the cartilage means that less damage to the ear will occur, and there will be less discomfort for the animal and few problems with the healing of the wound occurring. All these measures lead to a reduced risk of infection.

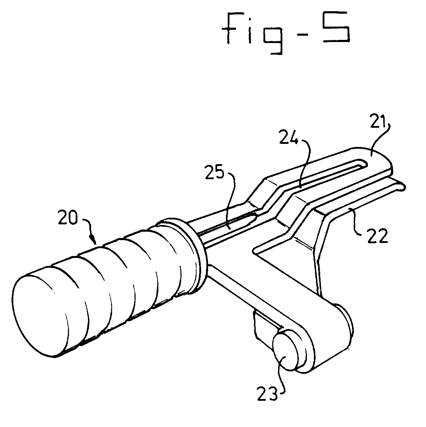

A further embodiment of the injector according to the invention is shown in Fig. 5. It is shown in its entirety by 20 and comprises a support 21 and an auxiliary support 22. Unlike the embodiment shown above, these are laterally hinged together at 23. A slit 24 is present in support 21, through which slit the conveyance of transponder 25 can be observed.

Although the invention is described above with reference to a preferred embodiment, it must be understood that numerous modifications can be made thereto. For example, it is possible to insert the transponder above the cartilage of the ear. It is also possible to modify the injector in such a way that it is suitable for the insertion of transponders in other supple parts of the body, for example the space between the lower jaw rami, and folds of the skin. Of course, the surfaces of the injector which position and deform the supple part of the body will be adapted accordingly in this case. All these modifications are considered to lie within the scope of the appended claims.

## Claims

1. Injector for the insertion of an object such as a transponder into a part of the body, comprising a feed device (7) for said object (6; 25), a piercing device (4) for said part of the body, and guide means (10, 11; 21, 22) for positioning the injector and the part of the body relative to each other, wherein said guide means (10, 11; 21, 22) comprises a support (10; 21) with a surface (12) for resting against the part of the body,
characterized in that
said surface (12) is composed of two substantially parallel parts lying at a distance from each other, between which a transition part is provided, the piercing device (4) for the supple part of the body being inserted near said transition part and in that said guide means comprises further an auxiliary support (11, 22) with an auxiliary surface (13) of a shape corresponding substantially to the shape of the surface (12) of the support (10; 21) for clamping and retaining the part of the body between the surface (12) of the support (10, 21) and the auxiliary surface (13) of the auxiliary support (11, 22).

2. Injector according to claim 1, in which the support (10; 21) and auxiliary support (11; 22) are hingedly connected to each other.

3. Injector according to claim 1 or 2, in which means are present for driving the support (10; 21) and auxiliary support (11; 22) towards each other.

4. Injector according to any of the preceding claims, in which the piercing device (4) is fitted on the object (6; 25) to be inserted.

## Patentansprüche

1. Injektionsvorrichtung zum Einführen eines Objekts, z.B. eines Transponders, in einen Körperteil mit einer Vorrichtung (7) zum Zuführen des Objekts (6; 25), einer Vorrichtung (4) zum Durchstechen des Körperteils und Führungsmitteln (10, 11; 21, 22), um die Injektionsvorrichtung und den Körperteil relativ zueinander zu positionieren, wobei die Führungsmittel (10, 11; 21, 22) ein Halteteil (10; 21) mit einer Fläche (12) umfassen, die dazu dient, an dem Körperteil anzuliegen,
dadurch gekennzeichnet, daß
die Fläche (12) aus zwei im wesentlichen parallelen Teilen besteht, die in einem Abstand zueinander angeordnet sind und zwischen denen ein Übergangsteil vorgesehen ist, wobei die Vorrichtung (4) zum Durchstechen des weichen Körperteils in der Nähe des Übergangsteils eingeführt wird, und dadurch, daß das Führungsmittel darüber hinaus ein Unterstützungshalteteil (11; 22) mit einer Unterstützungsfläche (13) aufweist, deren Form im wesentlichen der Form der Fläche (12) des Halteteils (10; 21) entspricht, und das dazu dient, den Körperteil zwischen die Fläche (12) des Halteteils (10, 21) und die Unterstützungsfläche (13) des Unterstützunghalteteils (11; 22) zu klemmen und zwischen diesen Flächen festzuhalten.

2. Injektionsvorrichtung nach Anspruch 1, bei der das Halteteil (10; 21) und das Unterstützungshalteteil über ein Gelenk miteinander verbunden sind.

3. Injektionsvorrichtung nach Anspruch 1 oder 2, bei der Mittel vorgesehen sind, die dazu dienen, das Kalteteil (10; 21) und das Unterstützungshalteteil (11; 22) gegeneinander zu treiben.

4. Injektionsvorrichtung nach einem der vorhergehenden Ansprüche, bei der die Vorrichtung (4) zum Durchstechen auf das einzusetzende Objekt (6; 25) aufgesetzt ist.

## Revendications

1. Injecteur pour l'insertion d'un objet tel qu'un répondeur dans une partie d'un corps, comprenant un dispositif d'amenée (7) pour ledit objet (6;25), un dispositif de perçage (4) pour ladite partie du corps, et des moyens de guidage (10,11;21,22) pour positionner l'injecteur et la partie du corps l'un par rapport à l'autre, dans lequel lesdits moyens de guidage (10,11;21,22) comportent un support (10;21) avec une surface (12) pour reposer contre la partie du corps, caractérisé en ce que ladite surface (12) est composée de deux parties sensiblement parallèles s'étendant à une distance l'une de l'autre, entre lesquelles est prévue une partie de transition, le dispositif de perçage (4) pour la partie souple du corps étant inséré près de ladite partie de transition et en ce que les moyens de guidage comportent en outre un support auxiliaire (11,22) avec une surface auxiliaire (13) de forme correspondant sensiblement à celle de la surface (12) du support (10;21) pour serrer et retenir la partie du corps entre la surface (12) du support (10,21) et la surface auxiliaire (13) du support auxiliaire (11,22).

2. Injecteur selon la revendication 1, dans lequel le support (10;21) et le support auxiliaire (11;22) sont connectés de manière pivotante l'un à l'autre.

3. Injecteur selon l'une des revendications 1 et 2, dans lequel il est prévu des moyens pour mener le support (10;21) et le support auxiliaire (11;22) l'un vers l'autre.

4. Injecteur selon l'une quelconque des revendications précédentes, dans lequel le dispositif de perçage (4) est monté sur l'objet (6;25) devant être inséré.
